# FASCICULE DE BREVET EUROPEEN

(11) **EP 1 951 341 B1**
(45) Date de publication et mention de la délivrance du brevet: **02.03.2011**
(21) Numéro de dépôt: 06831088.7
(22) Date de dépôt: 07.11.2006
(51) Int. Cl.: A61M 5/168, G01P 13/00

(54) **PROCEDE DE DETECTION D'OCCLUSION D'UNE TUBULURE POUR APPAREIL D'ADMINISTRATION DE LIQUIDES PHYSIOLOGIQUES**
VERFAHREN ZUR ENTDECKUNG EINER SCHLAUCHVERSTOPFUNG FÜR EINE VORRICHTUNG ZUR VERABREICHUNG PHYSIOLOGISCHER FLÜSSIGKEITEN
METHOD FOR DETECTING THE OCCLUSION OF A TUBING FOR A DEVICE FOR ADMINISTERING PHYSIOLOGICAL LIQUIDS

(30) Priorité: 16.11.2005 FR 0511610; 06.11.2006 FR 0609671
(43) Date de publication de la demande: 06.08.2008
(73) Titulaire: Eleph'ent Technology SA, 77420 Champs sur Marne (FR)
(72) Inventeur: RENAUX, Serge, F-34600 Les Aires (FR); LOUBERSSAC, Raymond, F-83700 SAINT-RAPHAEL (FR)
(74) Mandataire: Gosse, Michel
(86) Numéro de dépôt international: PCT/FR2006/002488
(87) Numéro de publication internationale: WO 2007/057542

(56) Documents cités:
- WO-A-98/04301
- FR-A- 2 495 475
- GB-A- 2 176 595

## Description

### DOMAINE DE L'INVENTION

La présente invention se rapporte à un procédé de détection d'occlusion et d'arrachement d'une tubulure destinée à l'administration, par voie entérale, de liquides physiologiques, ainsi qu'à l'appareil destiné à la mise en oeuvre dudit procédé.

### ARRIERE PLAN TECHNOLOGIQUE

On connaît des appareillages d'administration, par voie entérale, de liquides physiologiques du genre comprenant :
a) une pompe, notamment du type péristaltique rotatif ou linéaire ;
b) une tubulure destinée à l'administration desdits liquides ;
c) un dispositif de détection d'occlusion, constitué :
   - d'une zone d'entrée de fluide reliée à une première partie de ladite tubulure d'administration et une zone de sortie de fluide reliée à une seconde partie de ladite tubulure ;
   - d'une zone de détection d'occlusion, disposée selon une direction sensiblement perpendiculaire à la direction d'écoulement dudit fluide, appartenant à une structure tubulaire trivoie, agencée de sorte que ledit fluide pénètre dans ladite zone de détection en cas d'occlusion de ladite seconde partie de ladite tubulure ;
   - d'un moyen de détection de la présence d'un fluide dans ladite zone de détection, constitué d'un émetteur d'une onde électromagnétique et d'un récepteur de ladite onde électromagnétique ;
d) un calculateur programmé pour mesurer l'intensité de l'onde électromagnétique reçue au niveau dudit récepteur afin de détecter la présence dudit fluide dans ladite zone de détection lorsque l'intensité de ladite onde électromagnétique atteint un seuil prédéfini et de déclencher, dans ce dernier, cas une alarme.

De tels appareillages sont essentiellement décrits dans les brevets FR2945475 et GB2176595.

Ils présentent tous les principaux inconvénients liés à :
a) la détection d'occlusion après que le fluide ait pénétré dans la zone de détection suite à une première occlusion : en effet, les caractéristiques du rayonnement reçu au niveau du moyen de détection sont perturbés puisque la zone de détection a été mouillée par le fluide qui engendre une modification de la tension superficielle à cause des traces qui peuvent stagner en face du moyen de détection obligeant le changement du tube à chaque occlusion ce qui n'est pas fonctionnel ;
b) à l'arrachement de la structure tubulaire trivoie ainsi que de la deuxième partie de la tubulure d'administration, ce qui a pour effet de rendre inopérante la pompe ;
c) au manque de moyens de réglage, autre que celui consistant à déplacer l'ensemble émetteur et récepteur, pour faire varier le seuil de déclenchement de l'alarme.

### RESUME DE L'INVENTION

L'invention vise à concevoir un procédé et à réaliser un appareil pour sa mise en oeuvre qui élimine les inconvénients susmentionnés.

Pour ce faire, le procédé selon l'invention se caractérise essentiellement en ce qu'il consiste :
- dans le cas d'occlusions répétitives, à programmer le calculateur pour mesurer systématiquement et automatiquement l'intensité de l'onde reçue par le récepteur au moment de chaque occlusion et utiliser cette valeur comme valeur de référence pour déterminer le nouveau seuil d'intensité de détection à utiliser lors de chaque démarrage ou redémarrage, notamment pour prendre en compte les traces de liquide pouvant stagner et éviter ainsi d'avoir à changer la structure tubulaire trivoie à chaque détection d'occlusion ;
- pour s'adapter au type de liquide présent dans ladite tubulure, à programmer le calculateur pour mesurer, pendant un temps bien défini, la valeur de la tension entre les bornes du moyen de détection et établir cette valeur comme valeur de référence jusqu'au prochain changement de ladite tubulure ;
- pour modifier le seuil de déclenchement de l'alarme, à faire varier le volume d'air compressible contenu dans la zone de détection d'occlusion ;
- pour détecter l'arrachement de la structure tubulaire trivoie ainsi que la présence d'un fluide dans sa zone de détection, à utiliser un moyen de détection électromagnétique, notamment de type infrarouge ;
- pour détecter l'arrachement de la deuxième partie de la tubulure d'administration ainsi que la présence d'air dans celle-ci , à utiliser un moyen de détection électromagnétique, notamment de type ultrasonore.

Pour ce faire, l'appareil destiné à la mise en oeuvre dudit procédé se caractérise essentiellement en ce qu'il comprend :
- une pompe, une tubulure d'administration, une structure tubulaire trivoie pourvue d'une zone de détection de l'occlusion, un calculateur ;
- un moyen de détection électromagnétique de l'arrachement de ladite structure tubulaire trivoie et de la présence d'un fluide dans ladite zone de détection d'occlusion ;
- un moyen de détection électromagnétique de l'arrachement de la seconde partie de la tubulure de distribution et de la présence d'air dans celle-ci ;
- un bouchon, à hauteur réglable, ou interchangeable, apte à faire varier le volume d'air dans la zone de détection d'occlusion donc le seuil de déclenchement de l'alarme.

En l'absence d'occlusion, la pression du fluide est telle qu'il passe dans la tubulure sans pénétrer dans la zone de détection.

En cas d'occlusion de la tubulure, par exemple si le patient pince ou tord celle-ci ou tousse fortement mais dans ce cas il n'y a pas obstruction mécanique mais reflux temporaire au moment de la quinte de toux ou si la sonde est bouchée ou clampée, la pression va augmenter au sein du tube, et en réponse à cette augmentation de pression, le fluide va entrer dans la zone de détection. Lorsque le ménisque du liquide passera par exemple devant l'axe du seuil de détection, le récepteur électromagnétique enregistrera une modification de l'onde reçue permettant la détection de l'occlusion.

La zone de détection est dans ce cas sensiblement transparente audit rayonnement électromagnétique, ledit fluide étant sensiblement opaque audit rayonnement, de sorte que la détection d'occlusion peut être réalisée par la mesure de l'intensité au niveau dudit récepteur.

De préférence, lesdits moyens de détection définissent un axe de détection, ladite zone de détection traversant ledit axe de détection. De la sorte, le fluide peut être détecté lorsqu'il traverse l'axe de détection.

Ladite zone de détection peut être agencée selon une direction sensiblement perpendiculaire à la direction d'écoulement dudit fluide entre ladite zone d'entrée de fluide et ladite zone de sortie de fluide. De la sorte, on peut positionner facilement la zone de détection selon un axe de détection au niveau des moyens de raccordement. Afin que les mesures d'intensité du rayonnement puissent être précises même si la zone de détection a déjà été traversée par du fluide, le procédé peut comprendre en outre une étape d'étalonnage consistant à mesurer l'intensité dudit rayonnement électromagnétique reçu au niveau dudit récepteur de sorte à définir une valeur d'étalonnage de ladite intensité, ledit seuil prédéfini étant fonction de ladite valeur d'étalonnage de ladite intensité.

Dans le mode de fonctionnement normal, la pression dans le tampon d'air est plus forte que la pression à l'intérieur des zones d'entrée et de sortie de fluide à proximité de la zone de détection d'occlusion et le ménisque de fluide n'atteint pas l'axe de détection. Le rayonnement électromagnétique émis par l'émetteur est reçu par le récepteur après avoir traversé la zone de détection comprenant de l'air.

En cas d'occlusion de la partie aval de la tubulure, la pression au niveau de la zone de détection augmente et le ménisque du fluide pénètre dans ladite zone au-delà de l'axe de détection en comprimant l'air situé dans celle-ci. Dans ce cas, le rayonnement reçu par le récepteur est modifié.

L'émetteur et le récepteur sont par exemple associés à un calculateur, éventuellement intégré à la pompe, qui calcule l'intensité du rayonnement reçu par le récepteur en traitant le signal reçu.

Le calculateur est alors programmé pour émettre une alarme en cas de modification du rayonnement reçu au niveau du récepteur.

On notera que la programmation du calculateur dépend des caractéristiques du fluide distribué et du type de détection utilisée. Selon un mode de mise en oeuvre, on distribue par exemple des nutriments qui sont opaques au rayonnement électromagnétique choisi et on utilise un couple émetteur-récepteur pouvant émettre et détecter ledit rayonnement. Dans ce cas, lorsque le ménisque du fluide pénètre jusqu'à l'axe de détection de la zone de détection, le signal émis par l'émetteur est bloqué par les nutriments et n'est plus reçu par le récepteur. Le calculateur détecte alors cette modification du signal reçu et déclenche l'alarme.

### PRESENTATION DES FIGURES

Les caractéristiques et les avantages de l'invention vont apparaître plus clairement à la lecture de la description détaillée qui suit d'un mode de réalisation préféré de celle-ci donné à titre d'exemple non limitatif et représenté aux dessins annexés.

Sur ces dessins :

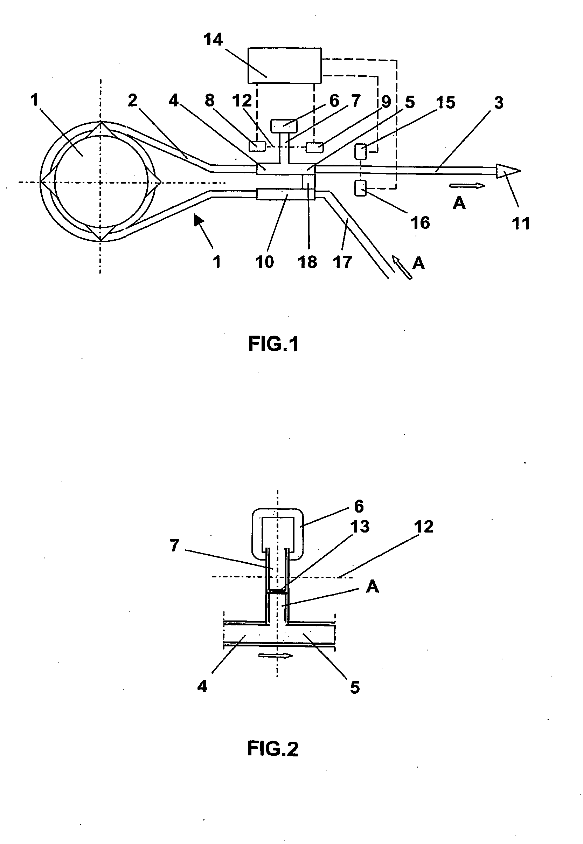

- la figure 1 est un schéma d'ensemble de l'appareil selon l'invention ;
- la figure 2 est une vue de détail en coupe de la structure tubulaire trivoie et du bouchon obturant et délimitant le volume d'air compressible de la zone de détection d'occlusion ;

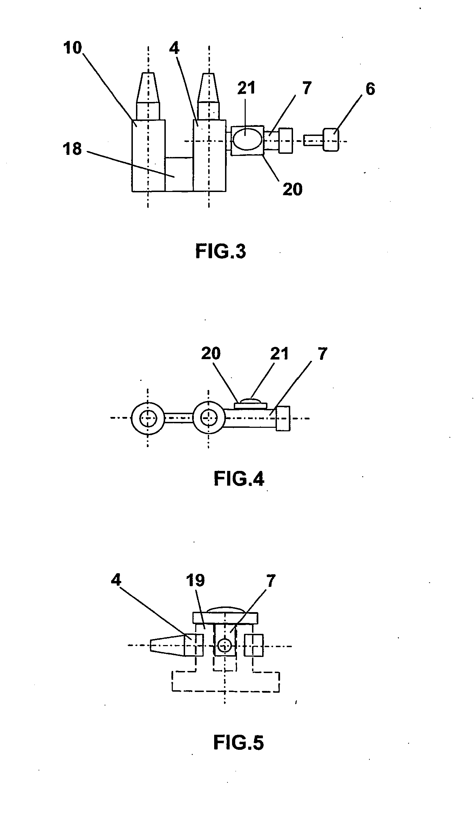

- les figures 3 et 4 sont respectivement des vues de dessus et frontale du dispositif de détection d'occlusion mettant en évidence les ailettes de positionnement ;
- la figure 5 est une vue de profil dudit dispositif de détection d'occlusion mettant en évidence la forme carrée de la zone d'occlusion et son positionnement entre les fourches du rayonnement électromagnétique.

### DESCRIPTION DETAILLEE DE L'INVENTION

L'appareil représenté aux figures, destiné à l'administration, par voie entérale, de liquides physiologiques (A), notamment nutritionnels, médicamenteux ou hybriques, comporte essentiellement :
a) une pompe (1), ou un système de pompage, notamment du type péristaltique rotatif ou linéaire, à débit fixe ou programmable ;
b) une tubulure d'administration (2,3) ;
c) un dispositif de détection d'occlusion (4,5,7) constitué :
   - d'une zone d'entrée de fluide (4) reliée à une première partie (2) de la tubulure d'administration et d'une zone de sortie de fluide (5) reliée à une seconde partie (3) de ladite tubulure ;
   - d'une zone (7), destinée à la détection d'une occlusion, disposée selon une direction sensiblement orthogonale à la direction d'écoulement dudit fluide, formant une structure tubulaire trivoie (4,5,7), agencée de sorte que ledit fluide pénètre dans ladite zone de détection (7) en cas d'occlusion de ladite seconde partie (3) de ladite tubulure d'administration (2,3) ;
   - d'un moyen de détection (8, 9) de la présence d'un fluide (A) dans ladite zone de détection (7), constitué d'un émetteur (8) d'une onde électromagnétique et d'un récepteur (9) de ladite onde électromagnétique définissant un axe de détection (12) ; ledit fluide étant sensiblement opaque à ladite onde électromagnétique ;
d) un calculateur (14) programmé pour mesurer l'intensité de ladite onde électromagnétique reçue au niveau dudit récepteur afin de détecter la présence dudit fluide dans ladite zone de détection lorsque l'intensité de ladite onde électromagnétique atteint un seuil prédéfini et de déclencher, dans ce dernier cas, une alarme.

Le procédé de détection d'occlusion et d'arrachement de la tubulure (2,3), auto-paramètrable, consiste :
- dans le cas d'occlusions répétitives, à programmer le calculateur (14) pour mesurer systématiquement et automatiquement l'intensité de l'onde reçue par le récepteur (9) au moment de chaque occlusion et utiliser cette nouvelle valeur comme valeur de référence pour déterminer le nouveau seuil d'intensité de détection à utiliser lors de chaque démarrage ou redémarrage ;
- pour s'adapter au type de liquide présent dans la structure tubulaire trivoie (4,5,7), à programmer le calculateur (14) pour mesurer, pendant un temps prédéfini, la valeur de la tension entre les bornes du moyen de détection et établir cette valeur comme valeur de référence jusqu'au prochain changement de ladite structure tubulaire ;
- pour modifier le seuil de déclenchement de l'alarme, à faire varier le volume d'air compressible contenu dans la zone de détection d'occlusion (7) ;
- à utiliser le moyen de détection électromagnétique (8,9), notamment de type infrarouge, pour détecter également la présence, dans sa zone de fixation, de la structure tubulaire trivoie (4,5,7) ;
- pour détecter la présence, dans sa zone de fixation, de la seconde partie (3) de la tubulure d'administration (2,3) ainsi que la présence d'air dans celle-ci, à utiliser un moyen (15,16), constitué d'un émetteur (15) d'une onde électromagnétique, notamment de type ultrasonore, et d'un récepteur (16) de ladite onde électromagnétique ;
- à programmer le calculateur (14) pour ne déclencher l'alarme qu'au bout d'un temps prédéfini par rapport à la détection du seuil de déclenchement.

L'appareil représenté comporte également :
- une tubulure d'arrivée (17) en provenance du réservoir contenant le liquide à administrer ;
- un manchon (10) permettant de relier ladite tubulure à l'extrémité de la tubulure (2) qui passe dans la pompe (1) ;
- un raccord (11) pour sonde destinée à l'administration entérale ;
- en partie supérieure de la zone de détection d'occlusion (7), un bouchon (6), à hauteur réglable (notamment par vissage), ou interchangeable, apte à faire varier le volume d'air dans ladite zone de détection d'occlusion ;
- une paroi (18) apte à relier la structure tubulaire trivoie (4,5,7) au manchon (10) de manière à former une seule pièce.

Le ménisque de la colonne de liquide dans la zone de détection d'occlusion (7), porte le repère (13).

Le dispositif de détection comprenant la zone de détection (7), la zone d'entrée de fluide (4) et la zone de sortie de fluide (5), peut être soit raccordé à une tubulure, soit formé d'une seule pièce au sein de la tubulure elle-même.

Il comprend des moyens de raccordement au niveau des zones d'entrée et de sortie qui sont adaptés aux tubulures utilisées et qui sont connus en soi dans le domaine médical.

Les tubulures utilisées en association avec le dispositif de détection d'occlusion sont par exemple des tubes en PVC, avec ou sans Phtalate, ou bien des tubes à base de matériau styrénique de type SEBS ou en silicone.

Le dispositif de détection d'occlusion peut être en matériau souple ou rigide, mais un matériau moulé rigide est préféré.

Le type de matériau utilisé pour le dispositif 1 de détection d'occlusion est par exemple un polymère rigide de type ABS, ou de 30 type Terlux (marque déposée) désignant un matériau de type MABS (Méthylméthacrylate - Acrylonitrile - Butadiène - Styrène). Le matériau utilisé pour le dispositif de détection d'occlusion est également choisi de sorte à pouvoir être collé aux tubulures en PVC.

L'homme du métier comprendra également que les moyens de détection (8) et (9) peuvent être positionnés au niveau de la pompe de distribution (1) de sorte à obtenir un dispositif compact. Dans ce cas, le calculateur (14) peut être le calculateur associé à ladite pompe. Il est également possible d'utiliser des moyens de détection séparés de la pompe et possédant un calculateur spécifique.

Les moyens de détection peuvent également avoir pour fonction de fixer la zone de détection (7) afin que celle-ci ne sorte pas de la zone de rayonnement. Des moyens de fixation différents des moyens de détection peuvent également être envisagés afin de clipser la zone de détection (7) entre la partie émettrice (8) et la partie réceptrice (9). En particulier, la zone de détection d'occlusion (7) peut posséder une forme carrée conçue pour s'adapter parfaitement entre les fourches (19) supportant l'émetteur (8) et le récepteur (9) de l'onde électromagnétique et comporter deux ailettes (20) aptes à se positionner en appui sur lesdits émetteur et récepteur afin d'assurer son placement correct sur le trajet de ladite onde et un moyen (21) ayant un effet de loupe apte à vérifier visuellement que l'alarme générée par le calculateur (14) a pour origine une occlusion, un arrachement ou un mauvais positionnement de la tubulure trivoie (4,5,7). Enfin, on note que l'invention peut être utilisée avec tout type de fluide traversant une tubulure souple pour laquelle une occlusion est possible.

Les données du calculateur peuvent être enregistrées localement pour être exploitées en temps réel ou différé ou être transmises à distance à un organe central de traitement. Dans ce cas, les alarmes locales peuvent être inhibées.

Nous avons décrit la présente invention dans le cadre d'une utilisation médicale pour laquelle une occlusion peut avoir des conséquences très négatives, mais il est entendu que la présente invention ne dépend ni du fluide distribué dans la tubulure souple, ni du type de détection utilisé.

L'homme du métier est en effet apte à adapter les moyens de détection en fonction du fluide distribué et pourra choisir les émetteurs et les récepteurs dans tout le spectre électromagnétique, en fonction du fluide et des contraintes du domaine d'application.

## Revendications

1. Procédé de détection d'occlusion et d'arrachement d'une tubulure (2,3) appartenant à un appareil destiné à l'administration, par voie entérale, de liquides physiologiques (A) ; ledit appareil comprenant également :
a) une pompe (1), notamment du type péristaltique rotatif ou linéaire ;
b) un dispositif de détection d'occlusion (4,5,7) constitué :
- d'une zone d'entrée de fluide (4) reliée à une première partie (2) de ladite tubulure d'administration (2,3) et une zone de sortie de fluide (5) reliée à une seconde partie (3) de ladite tubulure ;
- d'une zone (7), destinée à la détection d'une occlusion, disposée selon une direction sensiblement perpendiculaire à la direction d'écoulement dudit fluide, formant une structure tubulaire trivoie (4,5,7), agencée de sorte que ledit fluide pénètre dans ladite zone de détection (7) en cas d'occlusion de ladite seconde partie (3) de la tubulure d'administration (2,3) ;
- d'un moyen (8,9) de détection de la présence d'un fluide (A) dans ladite zone de détection (7), constitué d'un émetteur (8) d'une onde électromagnétique et d'un récepteur (9) de ladite onde électromagnétique ;
c) un calculateur (14) programmé pour mesurer l'intensité de l'onde électromagnétique reçue au niveau dudit récepteur afin de détecter la présence dudit fluide dans ladite zone de détection lorsque l'intensité de ladite onde électromagnétique atteint un seuil prédéfini et de déclencher une alarme ;
**caractérisé en ce qu'**il consiste, dans le cas d'occlusions répétitives, à programmer le calculateur (14) pour mesurer systématiquement et automatiquement l'intensité de l'onde reçue par le récepteur (9) au moment de chaque occlusion et utiliser cette nouvelle valeur comme valeur de référence pour déterminer le nouveau seuil d'intensité de détection à utiliser lors de chaque démarrage ou redémarrage.

2. Procédé, selon la revendication 1, **caractérisé en ce qu'**il consiste, pour s'adapter au type de liquide présent dans la structure tubulaire trivoie (4,5,7), à programmer le calculateur (14) pour mesurer, pendant un temps prédéfini, la valeur de la tension entre les bornes du moyen de détection et établir cette valeur comme valeur de référence jusqu'au prochain changement de ladite structure tubulaire.

3. Procédé, selon la revendication 1, **caractérisé en ce qu'**il consiste, pour modifier le seuil de déclenchement de l'alarme, à faire varier le volume d'air compressible contenu dans la zone de détection d'occlusion (7).

4. Procédé, selon la revendication 1, **caractérisé en ce qu'**il consiste à utiliser le moyen de détection électromagnétique (8,9) pour détecter également la présence, dans sa zone de fixation, de la structure tubulaire trivoie (4,5,7).

5. Procédé, selon la revendication 1, **caractérisé en ce qu'**il consiste, pour détecter la présence, dans sa zone de fixation, de la seconde partie (3) de la tubulure d'administration (2,3) ainsi que la présence d'air dans celle-ci, à utiliser un moyen (15,16), constitué d'un émetteur (15) d'une onde électromagnétique et d'un récepteur (16) de ladite onde électromagnétique.

6. Procédé, selon la revendication 4, **caractérisé en ce qu'**il consiste à utiliser comme onde électromagnétique, une onde infrarouge.

7. Procédé, selon la revendication 5, **caractérisé en ce qu'**il consiste à utiliser comme onde électromagnétique, une onde ultrasonore.

8. Appareil pour la mise en oeuvre du procédé selon l'une quelconque des revendications précédentes, **caractérisé en ce qu'**il comprend une pompe (1) ; une tubulure d'administration (2,3) ; une structure tubulaire trivoie (4,5,7) pourvue d'une zone de détection d'occlusion (7) ; un calculateur (14) ; un moyen (8,9), constitué d'un émetteur (8) d'une onde électromagnétique et d'un récepteur (9) de ladite onde électromagnétique, apte à détecter la présence d'un fluide dans ladite zone de détection d'occlusion et l'arrachement de ladite structure tubulaire trivoie ; un moyen (15,16), constitué d'un émetteur (15) d'une onde électromagnétique et d'un récepteur (16) de ladite onde électromagnétique, apte à détecter la présence d'air dans la deuxième partie (3) de la tubulure d'administration (2,3) et l'arrachement de celle-ci ; **caractérisé en ce que** la zone de détection d'occlusion (7) comporte, en partie supérieure, un bouchon (6), à hauteur réglable, ou interchangeable, apte à faire varier le volume d'air dans la zone de détection d'occlusion (7).

9. Appareil, selon la revendication 8, **caractérisé en ce que** la zone de détection d'occlusion (7) possède une forme carrée conçue pour s'adapter parfaitement entre les fourches (19) supportant l'émetteur (8) et le récepteur (9) de l'onde électromagnétique et comporter deux ailettes (20) aptes à se positionner en appui sur lesdits émetteur et récepteur afin d'assurer son placement correct sur le trajet de ladite onde et un moyen (21) ayant un effet de loupe apte à vérifier visuellement que l'alarme générée par le calculateur (14) a pour origine une occlusion, un arrachement ou un mauvais positionnement de la tubulure trivoie (4,5,7).

## Claims

1. Method for detecting obstruction and extraction of a tubing (2,3) belonging to an apparatus for administering, by the enteral route, physiological fluids (A); said apparatus further comprising:
a) a pump (1), particularly a linear or rotary peristaltic pump;
b) a device for detecting obstruction (4,5,7) consisting of:
- a fluid inlet zone (4) connected to a first portion (2) of said administration tubing (2,3) and a fluid outlet zone (5) connected to a second portion (3) of said tubing;
- a zone (7), for detecting an obstruction, arranged along a direction substantially perpendicular to the flow direction of said fluid, forming a three-way tubular structure (4,5,7), arranged such that said fluid enters said detection zone (7) in the event of obstruction of said second portion (3) of the administration tubing (2,3);
- means (8,9) for detecting the presence of a fluid (A) in said detection zone (7), consisting of a transmitter (8) of an electromagnetic wave and a receiver (9) of said electromagnetic wave;
c) a computer (14) programmed to measure the intensity of the electromagnetic wave received on said receiver in order to detect the presence of said fluid in said detection zone when the intensity of said electromagnetic wave reaches a predefined threshold and activate an alarm;
**characterised in that** it consists, in the event of repetitive obstructions, of programming the computer (14) to measure, systematically and automatically, the intensity of the wave received by the receiver (9) as each obstruction occurs and using these new value as a reference value for determining the new detection intensity threshold to use following each start or restart.

2. Method, according to claim 1, **characterised in that** it consists, to adapt to the type of fluid present in the three-way tubular structure (4,5,7), of programming the computer (14) to measure, for a predefined time, the voltage value between the terminals of the detection means and set said value as the reference value until the next replacement of said tubular structure.

3. Method, according to claim 1, **characterised in that** it consists, to modify the alarm activation threshold, of varying the compressible air volume contained in the obstruction detection zone (7).

4. Method, according to claim 1, **characterised in that** it consists of using the electromagnetic detection means (8,9) for also detecting to the presence, in the attachment zone, of the three-way tubular structure (4,5,7).

5. Method, according to claim 1, **characterised in that** it consists, to detect the presence, in the attachment zone thereof, of the second portion (3) of the administration tube (2,3) and the presence of air therein, using means (15,16), consisting of a transmitter (15) of an electromagnetic wave and a receiver (16) of said electromagnetic wave.

6. Method, according to claim 4, **characterised in that** it consists of using an infrared wave as the electromagnetic wave.

7. Method according to claim 5, **characterised in that** it consists of using an infrared wave as the electromagnetic wave.

8. Apparatus for applying the method according to any of the above claims, **characterised in that** it comprises a pump (1); an administration tubing (2,3); a three-way tubular structure (4,5,7) provided with an obstruction detection zone (7); a computer (14); means (8,9), consisting of a transmitter (8) of an electromagnetic wave and a receiver (9) of said electromagnetic wave, suitable for detecting the presence of a fluid in said obstruction detection zone and the extraction of said three-way tubular structure; means (15,16), consisting of a transmitter (15) of an electromagnetic wave and a receiver (16) of said electromagnetic wave, suitable for detecting the presence of air in the second portion (3) of the administration tubing (2,3) and the extraction thereof;
**characterised in that** the obstruction detection zone (7) comprises, in the upper portion, a height-adjustable or interchangeable plug (6), suitable for varying the volume of air in the obstruction detection zone (7).

9. Apparatus, according to claim 8, **characterised in that** the obstruction detection zone (7) has a square shape designed to fit perfectly between the forks (19) supporting the transmitter (8) and the receiver (9) of the electromagnetic waves and comprise two ribs (20) suitable for being positioned bearing on said transmitter and receiver to ensure the correct positioning thereof on the trajectory of said wave and means (21) having a magnifier effect suitable for visually checking that the alarm generated by the computer (14) has been caused by an obstruction, extraction or incorrect positioning of the three-way tubing (4,5,7).

## Patentansprüche

1. Verfahren zur Erkennung der Okklusion und des Losreißens eines Rohres (2,3) von einem Gerät, das zur Verabreichung von physiologischen Flüssigkeiten (A) auf enteralem Weg vorgesehen ist; wobei das Gerät ebenfalls Folgendes umfasst:
a) eine Pumpe (1), insbesondere des Typs der rotierenden oder linearen Peristaltikpumpe;
b) eine Vorrichtung zur Erkennung der Okklusion (4,5,7), die sich aus Folgendem zusammen setzt:
- eine Flüssigkeitseintrittszone (4), die mit einem ersten Teil (2) des Verabreichungsrohres (2,3) verbunden ist, und eine Flüssigkeitsaustrittszone (5), die mit einem zweiten Teil (3) des Rohres verbunden ist;
- eine Zone (7), die zur Erkennung einer Okklusion vorgesehen ist, die nach einer fast senkrechten Richtung zur Stromrichtung der Flüssigkeit angeordnet ist, die eine 3-Wege-Rohrstruktur (4,5,7) bildet, die derart angeordnet ist, dass die Flüssigkeit im Fall der Okklusion des zweiten Teils (3) des Verabreichungsrohres (2,3) in die Erkennungszone (7) eindringt;
- ein Mittel (8,9) zur Erkennung der Anwesenheit einer Flüssigkeit (A) in der Erkennungszone (7), das sich aus einem Sender (8) einer elektromagnetischen Welle und einem Empfänger (9) der elektromagnetische Welle zusammen setzt;
c) ein Rechner (14), der programmiert ist, um die Intensität der elektromagnetischen Welle zu messen, die beim Empfänger empfangen wird, um die Anwesenheit der Flüssigkeit in der Erkennungszone zu erkennen, wenn die Intensität der elektromagnetischen Welle einen vorbestimmten Grenzwert erreicht, und um einen Alarm auszulösen;
**dadurch gekennzeichnet, dass** es im Fall der wiederholten Okklusionen darin besteht, den Rechner (14) zu programmieren, um systematisch und automatisch die Intensität der Welle, die vom Empfänger (9) zum Zeitpunkt von jeder Okklusion empfangen wird, zu messen, und um diesen neuen Wert als Bezugswert zu verwenden, um den neuen Grenzwert der Erkennungsintensität zu bestimmen, der bei jedem Start oder Neustart zu verwenden ist.

2. Verfahren nach Anspruch 1, **dadurch gekennzeichnet, dass** es darin besteht, zur Anpassung an den Typ der Flüssigkeit, die in der 3-Wege-Rohrstruktur (4,5,7) anwesend ist, den Rechner (14) zu programmieren, um während einer vorbestimmten Zeit den Wert der Spannung zwischen den Klemmen des Erkennungsmittels zu messen und diesen Wert als Bezugswert bis zur nächsten Änderung der Rohrstruktur festzulegen.

3. Verfahren nach Anspruch 1, **dadurch gekennzeichnet, dass** es darin besteht, zur Änderung des Grenzwertes zur Auslösung des Alarms, die Menge kompressibler Luft, die in der Okklusionserkennungszone (7) enthalten ist, variieren zu lassen.

4. Verfahren nach Anspruch 1, **dadurch gekennzeichnet, dass** es darin besteht, das Mittel zur elektromagnetischen Erkennung (8,9) zu verwenden, um ebenfalls die Anwesenheit in seiner Befestigungszone der 3-Wege-Rohrstruktur (4,5,7) zu erkennen.

5. Verfahren nach Anspruch 1, **dadurch gekennzeichnet, dass** es darin besteht, zur Erkennung der Anwesenheit, in der Befestigungszone, des zweiten Teils (3) des Verabreichungsrohres (2,3) sowie der Anwesenheit von Luft in ihr, ein Mittel (15,16) zu verwenden, das sich aus einem Sender (15) einer elektromagnetischen Welle und einem Empfänger (16) der elektromagnetischen Welle zusammen setzt.

6. Verfahren nach Anspruch 4, **dadurch gekennzeichnet, dass** es darin besteht, eine Infrarotwelle als elektromagnetische Welle zu verwenden.

7. Verfahren nach Anspruch 5, **dadurch gekennzeichnet, dass** es darin besteht, eine Ultraschallwelle als elektromagnetische Welle zu verwenden.

8. Gerät zur Umsetzung des Verfahrens nach einem der vorhergehenden Ansprüche, **dadurch gekennzeichnet, dass** es Folgendes umfasst: eine Pumpe (1); ein Verabreichungsrohr (2,3); eine 3-Wege-Rohrstruktur (4,5,7) mit einer Okklusionserkennungszone (7); einen Rechner (14); ein Mittel (8,9), das sich aus einem Sender (8) einer elektromagnetischen Welle und einem Empfänger (9) der elektromagnetischen Welle zusammen setzt, das fähig ist, die Anwesenheit einer Flüssigkeit in der Okklusionserkennungszone und des Losreißens der 3-Wege-Rohrstruktur zu erkennen; ein Mittel (15,16), das sich aus einem Sender (15) einer elektromagnetischen Welle und einem Empfänger (16) der elektromagnetischen Welle zusammen setzt, das fähig ist, die Anwesenheit von Luft im zweiten Teil (3) des Verabreichungsrohres (2,3) und dessen Losreißen zu erkennen;
**dadurch gekennzeichnet, dass** die Okklusionserkennungszone (7) im oberen Teil einen Stopfen (6) mit einstellbarer Höhe oder der austauschbar ist, umfasst, der fähig ist, die Luftmenge in der Okklusionserkennungszone (7) variieren zu lassen.

9. Gerät nach Anspruch 8, **dadurch gekennzeichnet, dass** die Okklusionserkennungszone (7) eine quadratische Form aufweist, die entwickelt wurde, um sich perfekt zwischen den Gabeln (19) anzupassen, die den Sender (8) und den Empfänger (9) der elektromagnetischen Welle tragen, und zwei Flügel (20) zu umfassen, die sich auf den Sender und den Empfänger stützen, um seine korrekte Lage auf der Strecke der Welle sicher zu stellen und ein Mittel (21) mit einem Lupeneffekt, das fähig ist, visuell zu prüfen, dass der vom Rechner (14) generierte Alarm auf einer Okklusion, einem Losreißen oder einer schlechten Lage des 3-Wege-Rohres (4,5,7) beruht.
